# EUROPEAN PATENT SPECIFICATION

(11) **EP 3 075 723 B1**
(45) Date of publication and mention of the grant of the patent: **24.05.2017**
(21) Application number: 15000922.3
(22) Date of filing: 30.03.2015
(51) Int. Cl.: C07C 229/36, A61K 9/70, A61K 31/198, A61K 31/216, A61P 25/16

(54) **HIGHLY SOLUBLE L-DOPA GLYCEROL ESTERS**
HÖCHLÖSLICHE L-DOPA-GLYCEROLESTER
ESTERS DE GLYCÉROL L-DOPA HAUTEMENT SOLUBLES

(43) Date of publication of application: 05.10.2016
(73) Proprietor: Berlirem GmbH, 15806 Zossen (DE)
(72) Inventor: Tack, Johannes, 13595 Berlin (DE); Wyrwa, Ralf, 07751 Rothenstein (DE); Laube, Thorsten, 06721 Osterfeld (DE); Schnabelrauch, Matthias, 07745 Jena (DE); Weisser, Jürgen, 99092 Erfurt (DE); Völkel, Christoph, 10318 Berlin (DE)
(74) Representative: Seuss, Thomas

(56) References cited:
- EP-A1- 0 287 341
- WO-A1-98/16208
- WO-A2-2012/079072
- US-A- 4 035 507

## Description

### Field of the invention

The invention describes the synthesis of levodopa (L-DOPA; L-3,4-dihydroxy-phenylalanin) glycerol esters by coupling glycerol or glycerol derivatives to the L-DOPA carboxyl group. The synthesis allows to produce L-DOPA derivatives which are highly soluble in water as well as aqueous and biocompatible liquids and have an improved hydrolytic stability in water or aqueous and biocompatible media for an application over several days. The invention helps producing L-DOPA substances for applications in the fields of medicine, biology and medical engineering as well as in the pharmaceutical industry.

### State-of-the-art

Parkinson's disease (PD) is among the neurological disorders a slowly progressing neurodegenerative disorder and belongs to the most frequent diseases of the central nervous system (CNS) [J. Jankovic, S. Fahn, Ann Intern Med., 93 (1980) 460 - 465]. The disease is triggered by degeneration of dopaminergic neurons in the *substantia nigra,* a structure in the midbrain that generates the neurotransmitter dopamine from L-DOPA by removing a carboxyl group from L-DOPA. A lack of dopamine will eventually result in considerable restrictions of the motor skills as well as symptoms in the psychological, sensory and vegetative areas.

The most effective therapy has proved to be the oral application of levodopa (L-DOPA) [O. Kofman, Can Med Assoc J., 104 (1971) 483 - 487], with L-DOPA being applied in the form of tablets. L-DOPA is a prodrug of the neurotransmitter dopamine that can cross the blood-brain barrier by an active amino acid transporter. The enzyme DOPA decarboxylase converts L-DOPA in the brain into the active form of dopamine. The life expectancy of Parkinson patients can be considerably extended with the oral L-Dopa therapy, provided complications due to illnesses are avoided, or the patients' quality of life can be improved. However, the therapeutic effect is lost after a maximum of five years, while oral L-DOPA therapy of longer duration may cause unintentional excess movements which must be attributed to the short action time of L-DOPA, which lasts a few hours only, in combination with an unphysiological pulsatile receptor stimulation [A. Barbeau, Can Med Assoc J., 101 (1969) 59 - 68; G. B. Stefano, K. J. Mantione, M. Králí 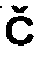 ková, R. Ptacek, H. Kuzelova, T. Esch, R. M. Kream, Med SciMonit., 18 (2012) 133 - 137].

On the other hand, dopamine agonists are used to stimulate the dopamine receptors (e.g. Bromocriptine, Ropinirol, Pramipexol) as further therapies for the treatment of Morbus Parkinson, also inhibitors of the catechol-O-methyl-transferase and of the monoamino oxidase that prevent the methylation or oxidation of L-DOPA to become ineffective metabolites by inhibiting these enzymes. Dopamine agonists as well as the enzyme inhibitors are usually used in combination with L-DOPA.

Although the therapy with L-DOPA and the biogenic amine dopamine derived from it is the best treatment, fluctuations in the effectiveness occurring in the late stage of the disease may affect the patients concerned and cause a sudden change between good mobility and severe immobility ("on-off phenomenon"). This medical risk must not be underestimated. In the current understanding, the cause of this severe complication must be attributed to the oral pulsatile L-DOPA administration.

For that reason, new approaches for applying the L-DOPA therapy are searched for. For example, the medicinal product Sinemet^{®}, a combination of levodopa and carbidopa (Carbidopa serves as a DOPA decarboxylase inhibitor) has been introduced [B. Boshes, Ann Intern Med., 94 (1981) 364 - 370]. Another example is DuoDopa^{®}, a product available in the market for some time now, which has made it possible for the first time ever to continuously administer L-DOPA. DuoDopa^{®} is a gel formulation (100 ml) of levodopa which will be administered continuously into the duodenum by way of percutaneous entero-gastrostomic surgery (PEG) [H. H. Fernandez, P. Odin, Curr Med Res Opin., 27 (2011), 907 - 919; L. Jain, R. Benko, S. Safranek, J Fam-Pract., 61 (2012) 106 -108; J. Sawek, A. Bogucki, NeurolNeurochir Pol., 44 (2010) 396 - 403]. The treatment results in a considerable improvement for the patient with regard to the motoric complications. It is, however, necessary, to administer relatively large quantities of liquid, because levodopa has only a low water solubility of 20 mg/ml (H. H. Fernandez, P. Odin, Curr Med Res Opin., 27 (2011). The treatment also requires surgery, i.e. the creation of an artificial intestinal access via percutaneous abdominal port, it may be necessary to replace this port from time to time due to a mechanical blockage. Since this is very cumbersome, DuoDopa^{®} is only be used in a limited number of patients.

Due to still unsolved problems concerning the L-DOPA treatment, improved therapeutic methods are urgently required, with suitable L-DOPA derivatives representing a major prerequisite, particularly in view of the solubility and bioavailability of the L-DOPA from these derivatives. Several prodrugs of L-DOPA are already described [A. Di Stefano, P. Sozio, L.S. Cerasa, A. lannitelli, Current Pharmaceutical Design, 2011, 17, 3482 - 3493] but only with respect to the oral dosage forms with the above described complications. A continuous approach to use prodrugs of L-DOPA with enhanced bioavailability is shown with respect to rectal administration [J.A. Fix, J. Alexander, M. Cortese, K. Engle, P. Leppert, A.J. Repta, Pharmaceutical Res., Vol 6, No. 6, 1989, p. 501-505].

EP 0287341 (priority 1988) describes a rectally absorbable form of L-DOPA where L-Dopa prodrugs are used. These used prodrugs are L-DOPA esters in which the ester components are substituted or unsubstituted mono-, di- or poly-hydroxyalkyl residues. The processes to synthesize the described substances are mentioned in US Patent Nos. 3,891,696 and 4,035,507 (priority 1973 and 1976).

These syntheses are unspecific with respect to reactions at all functional groups of L-DOPA and lead to substituted L-DOPA derivatives without free phenolic hydroxyl groups and with undefined numbers of substituents at the amino group. Preferably, polymers of L-DOPA derivatives are produced. These polymers / substituents are rather unfavorable for the intended physicochemical properties like high water solubility, fast enzymatic ester cleavage and sufficient stability of the compound in the solvent selected. The specific L-DOPA ester claimed in this application cannot be synthesized by using the methods described in the US patents mentioned. Therefore, the aim of the present invention was to define L-DOPA derivatives with the above mentioned preferred and favored properties and to produce them by specific synthesis steps which are new and inventive and not comparable to the syntheses used in the above mentioned US patents. WO012/079702 A2 discloses subcutaneously infusible L-DOPA prodrug compositions, in particular solutions comprising acid addition salts of L-DOPA ethyl ester (LDEE) such as LDEE.HCI. The presented synthesis includes the protection of the functional groups which need to remain unsubstituted and the selective esterification of the carboxyl group of L-DOPA. Furthermore, the synthesis is able to produce stereoselective compounds of L-DOPA esters if needed.

New derivatives can be potentially used in innovative application systems like e.g. patch pumps - known yet as insulin delivery devices for Diabetes Mellitus patients - for the continuous subcutaneous treatment of Morbus Parkinson with L-DOPA for the large majority of patients and thus to considerably improve the long-term therapy.

### Solution to the problem underlying the present invention

The present invention describes the synthesis of levodopa (L-DOPA; L-3,4-dihydroxyphenylalanin) glycerol esters by coupling glycerol or its derivatives to the L-DOPA carboxyl group, whereby the synthesis allows to produce L-DOPA substances which are highly soluble in water as well as in aqueous and biocompatible liquids and have an improved hydrolytic stability in water or aqueous liquids for an application over several days.

The present invention is therefore based on the task to produce L-DOPA derivatives that can be dissolved in water as well as in aqueous and biocompatible liquids in an easy and suitable way. The substances have an improved hydrolytic stability in water or aqueous and biocompatible media, and moreover, L-DOPA has to be released as biologically active component from the ester derivatives by hydrolytic or enzymatic cleavage after being administered to the organism.

This task will be accomplished in line with the invention in a suitable way by converting L-DOPA with glycerol or its derivatives into glycerol esters in an economical and easy way of synthesis. The new L-DOPA derivatives show considerable advantages compared with substances and forms of application used so far (see table 1 and 2). Due to their higher solubility the volume of the dosage form can be significantly reduced, resulting in a longer duration of the application and a higher patient compliance. Furthermore, due to their higher stability, the shelf life of these new derivatives shall be up to 3 years.

### Detailed description of the invention

The described features of the invention are substantiated by the following descriptions of exemplary embodiments which are presented in order to support the invention and are not intended to be limiting thereof.

Subject-matter of the present invention is a compound having the general Formula I 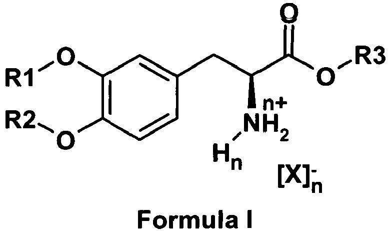
wherein [X]⁻ is a physiologically compatible anion,
wherein n is 0 or 1,
wherein R1 and R2 are, independently of each other, selected from the group comprising hydrogen, hydrogensulfate, phosphate, hydrogen phosphate, dihydrogen phosphate, benzoate, formate, acetate, propionate, butanoate, valerate, silyl, or
R1, R2 are both hydrogen phosphate, sulfate, methylene, isopropylidene,
wherein R3 represents a residue 1,3-dihydroxyprop-2-yl (+)-2,3-dihydroxyprop-1-yl, (-)-2,3-dihydroxyprop-1-yl or *rac*-2,3-dihydroxyprop-1-yl.

The physiologically compatible anion [X]⁻ is preferably selected from the group consisting of halogenide, sulfate, hydrogensulfate, phosphate, hydrogen phosphate, dihydrogen phosphate, carboxylate like benzoate, formate, acetate, propionate, butanoate, valerate, myristate, octoate, stearate, ascorbate, trifluoracetate, phosphonate, phosphoric acid ester, sulfonate (e.g. mesylate) or sulfuric acid ester (e.g. ethyl sulfate). The expert skilled in the art will appreciate that some anions carry two (e.g. sulfate) negative charges and will therefore be required in an amount of half a mole per mole of the cation. In cases in which the anion carries three negative charges (e.g. phosphate) only a third of a mole is required per mole of the cation.

In a preferred embodiment, subject-matter of the present invention is a compound of formula I, wherein [X]⁻ is Cl⁻, n is 1, R1 and R2 are both hydrogen.

In a specific embodiment,subject-matter of the present invention is a method of preparing a compound having general Formula I, comprising the steps of esterification of L-DOPA carrying protective groups of same structure for both the amino and phenolic L-DOPA functionalities with the OH-group of a (un)protected glycerol or its derivatives, and the selective removing of the protective groups subsequently. Subject-matter in another specific embodiment of the present invention is a method of preparing a compound having general Formula I, comprising the steps of coupling L-DOPA carrying protective groups of same structure for both amino and phenolic L-DOPA functionalities with an epoxide, whereby the ring of said epoxide is opened, and the selective removing of the protective group subsequently.

In a preferred embodiment, subject-matter of the present invention is a method of preparing a compound having general Formula I, wherein the L-DOPA ester is obtained by the following steps comprising:
(a) Introduction of suitable protective groups to protect the amino and phenolic functionalities of L-DOPA.
(b) Esterification of the carboxylic group of L-DOPA derivative containing protective groups of amino and phenolic functionalities.
(c) Subsequent cleavage of the protective groups from step (a).

As a preferred way of implementing the invention, L-DOPA will be converted in one pot synthesis into protected L-DOPA. Afterwards (un)protected glycerol is coupled by way of established esterification reactions or by opening of the epoxides rac.-glycidol or S-(-)-glycidol as well as R-(+)-glycidol enabling chiral glycerol esters. Following that, the protective groups are cleaved, so that the relevant L-DOPA glycerol ester can be obtained. The substances can be purified, if necessary, by way of recrystallization or column chromatography. For the process of further derivatization, corresponding L-DOPA glycerol esters will be converted into salts on basis of methods known to the experts.

In a more preferred embodiment, subject-matter of the present invention is a method of preparing a compound having general Formula I, wherein the protective groups are selected from the group comprising of tert.-butoxycarbonyl (Boc), silyl, carboxybenzyl (Cbz), benzyl, fluorenyloxycarbonyl (Fmoc), trityl, acetonide and others.

In another preferred embodiment, subject-matter of the present invention is a method of preparing a compound having general Formula I, wherein said compound can be purified by crystallization or preparative chromatographic methods after said preparation.

In another specific embodiment, subject-matter of the present invention is a method of preparing a compound having general Formula I, wherein said compound is produced regio-selectively.

Further subject-matter of the present invention is a compound having the general Formula I, wherein the solubility of said compound reaches more than 750 mg/ml in water or other aqueous and biocompatible liquids.

The compounds of the present application have a high solubility in solutions with a pH-value of 4-8 which correspond to the physiological range wherein the compounds should be used.

In a specific embodiment, subject-matter of the present invention is a compound having the general Formula I, wherein said compound has a high hydrolytic stability in acidic and alkaline environment (pH = 1 - 8.5).

The stability of the compounds of the present application is determined by hydrolysis according to Example 7.

In another embodiment, subject-matter of the invention is a compound having the general Formula I, wherein said compound will be cleaved by esterases according to Examples 8. In the context of the present invention, an esterase may for example be a carboxylesterase, preferably a human carboxylesterase, more preferably a recombinant human carboxylesterase, more preferably a recombinant human carboxylesterase 1, more preferably a recombinant human carboxylesterase 1 isoform c according to Example 9.

In another preferred embodiment, subject-matter of the present invention is a compound having the general Formula I for use in a method of treatment of neurological disorders.

In another preferred embodiment, subject-matter of the present invention is a compound having the general Formula I for use in a method of treatment of neurodegenerative disorders.

In another specific embodiment, subject-matter of the present invention is a compound having the general Formula I for use in a method of treatment of DOPA-receptor related disorders.

Further subject-matter of the present invention is a compound having the general Formula I for use in a method of treatment of Parkinson's disease.

In another embodiment, subject-matter of the present invention is a compound having the general Formula I for use in a method of treatment of neurodegenerative diseases, wherein said compound is formulated and administered as a suited dosage form commonly known in the pharmaceutical technology especially in form of a ready to use sterile solution or a sterile powder to be dissolved before application using excipients, process agents and buffer solutions or solvents for pharmaceutical use.

In another specific embodiment, subject-matter of the present invention is a compound having the general Formula I for use in a method of treatment of neurodegenerative diseases, wherein said compound is administered in dosages of between 100 mg and 700 mg per day over 3 to 5 days duration of a most favorable continuous application.

In another embodiment, subject-matter of the present invention is a compound of Formula I for use in a method of treatment of neurodegenerative diseases, wherein said compound is administered in dosages of between 100 mg and 700 mg per day over 3 to 5 days duration of a substantially continuous application, preferably parenteral administration.

In another more specific embodiment, subject-matter of the present invention is a composition comprising the compound having the general Formula I and one or more pharmaceutical acceptable excipients for pharmaceutical use.

In another preferred embodiment, subject-matter of the present invention is a composition comprising the compound having the general Formula I and one or more functional pharmaceutical excipients like antioxidants, stabilizer, antimicrobials for therapeutic use.

Neurological disorder is any disorder of the central nervous system. Dopamine-(receptor) related diseases may be for example Parkinson's disease, Schizophrenia, or Depression,
In the context of the present invention, a protective group, if not stated otherwise,is selected from the group comprising tert.-butoxycarbonyl (Boc), silyl, carboxybenzyl (Cbz), benzyl, fluorenyloxycarbonyl (Fmoc), trityl, acetonide and others
In mentioned herein, a halo or halogen group denotes fluorine, chlorine, bromine, or iodine; preferably chlorine.

The invention also provides a pharmaceutical composition comprising a compound of formula (I), in free form or in the form of pharmaceutically acceptable salts and physiologically functional derivatives, together with a pharmaceutically acceptable diluent or carrier therefore.

The pharmaceutical composition can be administered parenterally, e.g. in the form of injections or infusions.

In addition to the active compounds of formula (I), the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain two or more compounds of the formula (I) or their pharmacologically acceptable salts and also other therapeutically active substances.

Thus, the compounds of the present invention can be used in the form of one substance alone or in combination with other active compounds - for example with medicaments already known for the treatment of the aforementioned diseases, whereby in the latter case a favorable additive, amplifying effect is noticed.

To prepare the pharmaceutical preparations, pharmaceutically inert inorganic or organic excipients can be used. Suitable excipients for the production of injection solutions are, for example, water, alcohols, glycerol, polyols or vegetable oils.

Suitable salts for compounds of formula (I) according to this invention - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

According to expert's knowledge the compounds of formula (I) according to this invention as well as their salts may contain, e.g. when isolated in crystalline form, varying amounts of solvents. Included within the scope of the invention are therefore all solvates and in particular all hydrates of the compounds of formula (I) according to this invention as well as all solvates and in particular all hydrates of the salts of the compounds of formula (I) according to this invention. For instance, the mono-, di-, tri-, and tetrahydrates of formula (I) are encompassed.

The compounds according to the invention and medicinal drug products and compositions prepared therewith are generally suitable for the treatment of diseases which occur due to neurological disorders.

The compounds or corresponding medicinal drug products and compositions are particularly preferably suitable for the treatment of diseases caused by neurodegenerative disorders, in particular for the treatment of DOPA-receptor related disorders.

The compounds of the present invention or corresponding medicinal drug products and compositions are also useful for the treatment of Parkinson's disease.

The invention relates to the use of a composition according to the invention for the manufacture of a medicinal drug product and compositions.

The synthesis of alkyl esters, such as methyl, ethyl or 2-hydroxyethyl ester based on L-DOPA and the relevant types of alcohol in suitable solvents, with established protective group and coupling strategies being applied, has already been acknowledged; the solubility of the esters in water reaches up to 400 mg/ml for the ethyl ester hydrochloride salt (please see table 1).

Surprisingly and unexpectedly, it has been found that L-DOPA glycerol esters can be prepared regio- and stereoselectively and that they can have a higher hydrolytic stability in acidic and alkaline solutions (pH = 1 - 8.5) than simple alkylesters of the L-DOPA, although they can be enzymatically cleaved very fast. Furthermore, it has been found that the L-DOPA polyhydroxy esters have a very high aqueous solubility, i.e. equal to 2000 mg/ml. Due to this excellent solubility, these new esters are very well suited for applications in the therapy of Morbus Parkinson.

Table 1 shows the property differences of the hydrochloride salts of L-DOPA ethyl ester (Ethyl-Dopa_{*}HCl) and L-DOPA glycerol ester (Gly-Dopa_{*}HCl) with respect to important parameters for a suitable formulation.

**Table 1: Comparison of properties of Ethyl-Dopa*HCl and Gly-Dopa*HCl**

| **Parameter** | **Ethyl-Dopa*****HCl** | **Gly-Dopa*****HCl** |
|---|---|---|
| Aqueous solubility (g/ml)* | 0.4 | 2.0 |
| Stability (days at pH 7.4, Soerensen buffer)* | 2 | 14 |
| Time to complete hydrolysis (min) (*in vitro* enzymatic cleavage by a cell lysate)* | > 30 | 30 |
| Time to complete hydrolysis (min) (with carboxylesterase)* | 60 | > 60 |

| | | |
|---|---|---|
| * methods described in examples 6 - 9 | | |

The new L-DOPA glycerol esters show considerable advantages as compared with substances and forms of application used so far. Table 2 provides a comparison between DuoDopa^{®} and the possible system that consists of highly soluble L-DOPA derivatives and innovative application systems.

**Table 2: Comparison and assessment of DuoDopa^{®} and the highly soluble L-DOPA derivatives**

| **Parameter** | **DuoDopa^{®}** | **Highly soluble L-DOPA glycerol esters** | **Advantage of the L-DOPA glycerol esters** |
|---|---|---|---|
| Volume of the dosage form | 100 ml | 2 ml | ++ |
| Duration of the application | 1 day | 1 - 5 days | + |
| Patient compliance | low | high | ++ |
| Shelf life | 15 weeks | up to 3 years | ++ |
| Infection risk (through the type of application) | high | low | + |

| | | | |
|---|---|---|---|
| + = considerable advantage, ++ = outstanding advantage | | | |

Highly soluble L-DOPA glycerol esters are, for example,L-DOPA R-(+)-glycerol ester, L-DOPA R-(+)-glycerol ester hydrochloride, L-DOPA S-(-)-glycerol ester, L-DOPA S-(-)-glycerol ester hydrochloride, L-DOPA r*ac.*-glycerol ester, L-DOPA *rac*.-glycerol ester hydrochloride, L-DOPA 2-glycerol ester, L-DOPA 2-glycerol ester hydrochloride.

Patch pumps are electronic minipumps which are applied like an adhesive patch to the intact skin and allow after setting a s.c.- needle the continuous application for 1 or up to 5 days. The drug is contained in a small (2 - 5 ml) reservoir.

A preferred embodiment of the invention is the use of a pharmaceutical acceptable composition containing a compound according to formula I in a treatment of neurological disorders (such as Parkinson's disease) by continuous application of said composition via a patch pump.

L-DOPAglycerol esters to be suited for such an innovative dosage form have to show:
1. High solubility (at least 30 fold higher than L-DOPA)
2. Stability in water, aqueous and biocompatible media over several days
3. Fast hydrolysis by enzymatic cleavage after application

To apply L-DOPA continuously for 3 days using a daily dose of 500 mg a solubility of at least 750 to 1000 mg/ml is necessary to enable such an application. Neither L-DOPA itself has sufficient solubility to support such an innovative application nor the known alkanol ester of Levodopa especially L-DOPA methyl and ethyl ester. The solubility of L-DOPA is reported to be 20 mg/ml (H. H. Fernandez, P. Odin, Curr Med Res Opin., 27 (2011), the solubility of L-DOPA arginine or meglumin salts is reported to be *100 mg*/*ml at pH 9* (Patent WO 2012/066538 A1) and obviously not sufficient to enable the intended application by innovative patch pump technologies. The solubility of L-DOPA ethyl ester is reported to be higher in comparison to L-DOPA or solubility enhancing salts thereof amounting to 52 mg/ml at pH 8.2 for the free base (Patent US 6696600 B2, US 2003/0162832 A1) and 400 mg/ml in water for the hydrochloride salt (please see table 1). Furthermore, said alkanolester show unsufficient stability in aqueous solution and are not suited for the intended innovative application because of their hydrolysis products, in particular methanol.

### Examples

The following examples can be repeated with similar success by substituting the generically or specifically described reactants and/or operating conditions of this invention. It is believed than one skilled in the art can easily ascertain the essential characteristics of this invention and understands the Examples of the invention as exemplary. Thus the below examples are not limiting the subject-matter of the invention.

### EXAMPLES

The herewith disclosed Examples are meant to explain the invention in more detail without restricting it in any way.

### Example 1

### Synthesis of L-DOPA S-(-)-glycerol ester hydrochloride

### Variant 1

### Stage 1

### N,N-Dibenzyl-O3,O4-dibenzyl-L-DOPA hydrochloride

5 g (25.36 mmol) of L-DOPA were dissolved in 250 ml of acetone, followed by an addition of 17.52 g (126.78 mmol) of K₂CO₃, 195 mg (1.3 mmol) of potassium iodide and 12.05 ml (101.43 mmol) of benzyl bromide. The reaction mixture was heated for 96 h under reflux and the solvent was evaporated. Then 50 ml dioxane and 44 ml of 2 N NaOH were added and heated to reflux for 45 min. After cooling down to RT the solution was acidified with 2 N HCl to a pH of about 2 and kept at -18°C overnight. The precipitation was filtered off and dried in vacuum. The product was purified by recrystallisation from ethanol. A mixture of hydrochlorides of N-benzyl-03,04-dibenzyl-L-DOPA and N,N-dibenzyl-O3,O4-dibenzyl-L-DOPA was obtained. Both can be used for the following reactions.

### Stage 2

### N,N-Dibenzyl-O3,O4-dibenzyl-L-DOPA S-(-)-glycerol ester hydrochloride

1 g of the mixture of hydrochlorides of N-benzyl-O3,O4-dibenzyl-L-DOPA and N,N-dibenzyl-03,04-dibenzyl-L-DOPA was dissolved together with 0.55 g (2.14 mmol) of tetraethylammonium iodide in 50 ml of toluene and stirred for 5 min at 110 °C. Then 72 µl (1.08 mmol) of S-(-)-glycidol were added and the mixture was stirred for additional 20 min at 110°C. After each 20, 40 and 60 min additional 72 µl (1.08 mmol) S-(-)-glycidol were added. When the reaction was completed (DC control) the mixture was filtered and the solvent was evaporated. The product was purified by flash chromatography on silica gel with n-hexane/ethyl acetate (1:2). A mixture of hydrochlorides of N-benzyl-O3,O4-dibenzyl-L-DOPA glycerol ester and N,N-dibenzyl-03,04-dibenzyl-L-DOPA S-(-)-glycerol ester was obtained.

### Stage 3

### L-DOPA S-(-)-glycerol ester hydrochloride

1 g of a mixture of hydrochlorides of N-benzyl-O3,O4-dibenzyl-L-DOPA glycerol ester and N,N-dibenzyl-O3,O4-dibenzyl-L-DOPA glycerol ester was dissolved in 40 ml of MeOH/ethyl acetate (1:1). Pd/C (10 %) was added and the mixture was stirred in a autoclave at 3 - 25 bar H₂ pressure for several hours at RT. After the reaction was completed (DC control) the mixture was filtered and the solvent was evaporated. L-DOPA S-(-)-glycerol ester hydrochloride was obtained.

### Variant 2

### Stage 1

### N-Benzyl-O3,O4-dibenzyl-L-DOPA methyl ester

5 g (16.04 mmol) of L-DOPA methyl ester were dissolved in 250 ml of acetone, followed by an addition of 17.52 g (126.78 mmol) of K₂CO₃, 195 mg (1.3 mmol) of potassium iodide and 12.05 ml (101.43 mmol) of benzyl bromide. The reaction mixture was heated for 96 h under reflux and then the solvent was evaporated. The residue was dissolved in dichloromethane and extracted with water, with 5 % HCl, with water and with saturated NaCl solution. The combined organic extracts were dried with Na₂SO₄ and the solvent was evaporated. The product was purified by recrystallisation from ethanol. A mixture of hydrochlorides of N-benzyl-O3,O4-dibenzyl-L-DOPA methyl ester and N,N-dibenzyl-O3,O4-dibenzyl-L-DOPA methyl ester was obtained.

### Stage 2

### N-Benzyl-O3,O4-dibenzyl-L-DOPA hydrochloride

3 g of the mixture of hydrochlorides of N-benzyl-O3,O4-dibenzyl-L-DOPA methyl ester and N,N-dibenzyl-O3,O4-dibenzyl-L-DOPA methyl ester were dissolved in 40 ml of THF/Methanol (1:1), followed by addition of 9 ml of 3 M NaOH. The reaction was stirred 3 h at RT. After the reaction was completed the mixture is acidified with 4 N HCl to pH of about 3 and extracted with dichloromethane several times. The combined organic extracts were washed with water and saturated NaCl solution, dried with Na₂SO₄. Then the solvent was evaporated. A mixture of hydrochlorides of N-benzyl-O3,O4-dibenzyl-L-DOPA and N,N-dibenzyl-O3,O4-dibenzyl-L-DOPA was obtained. The product could be purified by flash chromatography on silica gel with n-hexane/ethyl acetate (1:2).

### Stage 3 and 4

### L-DOPA S-(-)-glycerol ester hydrochloride

To obtain L-DOPA glycerol ester the synthesis follows example 1, variant 1, stage 2 and 3.

### Example 2

### L-DOPA S-(-)-glycerol ester

200 mg of L-DOPA S-(-)-glycerol ester hydrochloride (example 1, variant 1, stage 3) was dissolved in 1 ml of water. To the solution 1 ml of saturated NaHCO₃ solution was added. The mixture was stirred for 10 min. Afterwards the water was removed and the residue extracted with ethyl acetate. After evaporation of the solvent L-DOPA S-(-)-glycerol ester was obtained.

### Example 3

### L-DOPA R-(+)-glycerol ester hydrochloride

### Stage 1

### N-benzyl-O3,O4-dibenzyl-L-DOPA R-(+)-glycerol ester hydrochloride

1 g of the mixture of hydrochlorides of N-benzyl-O3,O4-dibenzyl-L-DOPA and N,N-dibenzyl-03,04-dibenzyl-L-DOPA was dissolved together with 0.55 g (2.14 mmol) of tetraethylammonium iodide in 50 ml of toluene and stirred for 5 min at 110 °C. Then 72 µl (1.08 mmol) of R-(+)-glycidol were added and the mixture was stirred for additional 20 min at 110°C. After each 20, 40 and 60 min additional 72 µl (1.08 mmol) R-(+)-glycidol were added. When the reaction was completed (DC control) the mixture was filtered and the solvent was evaporated. The product was purified by flash chromatography on silica gel with n-hexane/ethyl acetate (1:2). A mixture of hydrochlorides of N-benzyl-O3,O4-dibenzyl-L-DOPA glycerol ester and N,N-dibenzyl-03,04-dibenzyl-L-DOPA R-(+)-glycerol ester was obtained.

### Stage 2

### L-DOPA R-(+)-glycerol ester hydrochloride

1 g of a mixture of hydrochlorides of N-benzyl-O3,O4-dibenzyl-L-DOPA R-(+)-glycerol ester and N,N-dibenzyl-O3,O4-dibenzyl-L-DOPA R-(+)-glycerol ester was dissolved in 40 ml of MeOH/ethyl acetate (1:1). Pd/C (10 %) was added and the mixture was stirred in a autoclave at 3 - 25 bar H₂ pressure for several hours at RT. After the reaction was completed (DC control) the mixture was filtered and the solvent was evaporated. L-DOPA R-(+)-glycerol ester hydrochloride was obtained.

### Example 4

### L-DOPA rac.-glycerol ester hydrochloride

### Stage 1

### N-Benzyl-O3,O4-dibenzyl-L-DOPA rac.-glycerol ester hydrochloride

1 g of the mixture of hydrochlorides of N-benzyl-O3,O4-dibenzyl-L-DOPA and N,N-dibenzyl-O3,O4-dibenzyl-L-DOPA was dissolved together with 0.55 g (2.14 mmol) of tetraethylammonium iodide in 50 ml of toluene and stirred for 5 min at 110 °C. Then 72 µl (1.08 mmol) of *rac*.-glycidol were added and the mixture was stirred for additional 20 min at 110°C. After each 20, 40 and 60 min additional 72 µl (1.08 mmol) *rac*.-glycidol were added. When the reaction was completed (DC control) the mixture was filtered and the solvent was evaporated. The product was purified by flash chromatography on silica gel with n-hexane/ethyl acetate (1:2). A mixture of hydrochlorides of N-benzyl-O3,O4-dibenzyl-L-DOPA *rac*.-glycerol ester and N,N-dibenzyl-03,04-dibenzyl-L-DOPA *rac*.-glycerol ester was obtained.

### Stage 2

### L-DOPA rac.-glycerol ester hydrochloride

1 g of a mixture of hydrochlorides of N-benzyl-O3,O4-dibenzyl-L-DOPA *rac*.-glycerol ester and N,N-dibenzyl-O3,O4-dibenzyl-L-DOPA *rac*.-glycerol ester was dissolved in 40 ml of MeOH/ethyl acetate (1:1). Pd/C (10 %) was added and the mixture was stirred in a autoclave at 3 - 25 bar H₂ pressure for several hours at RT. After the reaction was completed (DC control) the mixture was filtered and the solvent was evaporated. L-DOPA *rac*.-glycerol ester hydrochloride was obtained.

### Example 5

### L-DOPA sec.-glycerol ester

### Variant 1

### Stage 1

### N-benzyl-O3,O4-dibenzyl-L-DOPA sec.-glycerol ester

2.936 g (6.28 mmol) of the mixture of hydrochlorides of N-benzyl-03,04-dibenzyl-L-DOPA and N,N-dibenzyl-O3,O4-dibenzyl-L-DOPA were dissolved in 50 ml of ethyl acetate, 1.04 g (7.85 mmol) of 4,4-dimethyl-3,5-dioxacyclohexanol, 1.62 g (7.85 mmol) of DCC and 20 mg (0.17 mmol) of DMAP were added and the mixture was stirred overnight. Then the reaction mixture was filtered and the organic layer was treated with HCl, then washed with saturated sodium bicarbonate, water, dried with Na₂SO₄ and the solvent was evaporated. The crude product was purified by flash chromatography on silica gel with hexane/ethyl acetate (1:2). A mixture of N,N-dibenzyl-03,04-dibenzyl-L-DOPA sec.-glycerol ester and N-benzyl-O3,O4-dibenzyl-L-DOPA sec.-glycerol ester was obtained.

### Stage 2

### L-DOPA sec.-glycerol ester

1 g of the mixture of N,N-dibenzyl-O3,O4-dibenzyl-L-DOPA sec.-glycerol ester and N-benzyl-O3,O4-dibenzyl-L-DOPA sec.-glycerol esterwas dissolved in 40 ml of MeOH/ethyl acetate (1:1). Pd/C (10 %) was added and the mixture was stirred in an autoclave at 3 - 25 bar H₂ pressure for several hours at RT. After the reaction was completed (DC control) the mixture was filtered and the solvent was evaporated. L-DOPA sec.-glycerol ester was obtained.

### Variant 2

### Stage 1

### N-benzyl-03,04-dibenzyl-L-DOPA sec.-glycerol ester

2.936 g (6.28 mmol) of the mixture of hydrochlorides of N-benzyl-03,04-dibenzyl-L-DOPA and N,N-dibenzyl-O3,O4-dibenzyl-L-DOPA were dissolved in 50 ml of ethyl acetate, 1.16 g (7.85 mmol) of 2,2-dimethyl-1,3-dioxa-2-silacyclohexan-5-ol, 1.62 g (7.85 mmol) of DCC and 20 mg (0.17 mmol) of DMAP were added and the mixture was stirred overnight. Then the reaction mixture was filtered and the organic layer was treated with HCl, then washed with saturated sodium bicarbonate, water, dried with Na₂SO₄ and the solvent was evaporated. The crude product was purified by flash chromatography on silica gel with hexane/ethyl acetate (1:2). A mixture of N,N-dibenzyl-03,04-dibenzyl-L-DOPA sec.-glycerol ester andN-benzyl-O3,O4-dibenzyl-L-DOPA sec.-glycerol ester was obtained.

### Stage 2

### L-DOPA sec.-glycerol ester

1 g of the mixture of N,N-dibenzyl-O3,O4-dibenzyl-L-DOPA sec.-glycerol ester and N-benzyl-O3,O4-dibenzyl-L-DOPA sec.-glycerol ester was dissolved in 40 ml of MeOH/ethyl acetate (1:1). Pd/C (10 %) was added and the mixture was stirred in an autoclave at 3 - 25 bar H₂ pressure for several hours at RT. After the reaction was completed (DC control) the mixture was filtered and the solvent was evaporated. L-DOPA sec.-glycerol ester was obtained.

### Example 6

### Solubility

40 mg of lyophilized L-DOPA glycerol ester hydrochloride was dissolved in 20 µl of water resulting in a viscous liquid. The aqueous solubility of L-DOPA S-(-)-glycerol ester hydrochloride is ≥ 2 g/ml.

### Example 7

### Hydrolytic stability

Each 20 mg of lyophilized L-DOPA S-(-)-glycerol ester hydrochloride as well as L-DOPA ethyl ester hydrochloride were dissolved in 250 µl of buffer solution which was degassed for 5 min under argon. The pH values shown in table 3 were used.

**Table 3:Hydrolytic stability at selected pH values**

| **pH value** | **Buffer solution** |
|---|---|
| 1 | 0.1N hydrochloric acid |
| 4.5 | Soerensen (adjusted) |
| 7.4 | Soerensen (adjusted) |
| 8.5 | Soerensen (adjusted) |

The ester solutions were stored at 32 °C. After 1, 6, 8 h and 1, 2, 3, 6, 7, 14 d samples of 1 µl were investigated by thin-layer chromatography (TLC) using silica gel and ethyl acetate/MeOH/water (8:3:1).

L-DOPA S-(-)-glycerol ester is stable at all pH values over 14 days. In contrast L-DOPA ethyl ester will be cleaved within 2 d at pH of 7.4 and 8.5, within 14 d at pH = 4.5 but is stable at pH = 1 over measured time period.

### Example 8

### Enzymatic ester cleavage using cell lysate

About 1.64 x 10⁶ SD-1 cells (humane lymphoblastoide B cell line) were isolated by centrifugation. The cell pellet was resuspended in 400 µl of 12.5 mMTris/HCl solution of pH value of 7.4. Afterwards the cell suspention was sonificated under cooling (ice bath) over 30 s using Sonopuls HD2200 (Company Bandelin) at 50 % of power. Afterwards 100 µl of 5 mg/ml L-DOPA S-(-)-glycerol ester in water was added. The mixture was incubated for 30 and 180 min at 37 °C. The cleavage was controlled by TLC using silica gel and ethyl acetate/MeOH/water (8:3:1). The cleavage is almost complete after 30 min. After 180 min only traces of L-DOPA glycerol ester could be found. For purpose of comparison the L-DOPA ethyl ester was investigated under same conditions. The ethyl ester cleavage is lower compared to the glycerol ester.

### Example 9

### Enzymatic ester cleavage using isolated carboxylesterase

10 µl of 5 mg/ml L-DOPA S-(-)-glycerol ester in water was added to 35 µl of 14.3 mMTris/HCl solution of pH value of 7.4. Afterwards 5 µl carboxylesterase 1 (isoform c, human, recombinant (Sigma-Aldrich, Munich, Germany)) with activity ≥ 1000 units/mg protein (concentration: 5 mg/ml) was added. The mixture was incubated for 60 and 180 min at 37 °C. The cleavage was controlled by TLC using silica gel and ethyl acetate/MeOH/water (8:3:1). For purposes of comparison the L-DOPA ethyl ester was investigated under same conditions. The cleavage of ethyl ester is complete after 60 min. After 180 min only traces of L-DOPA glycerol ester could be found.

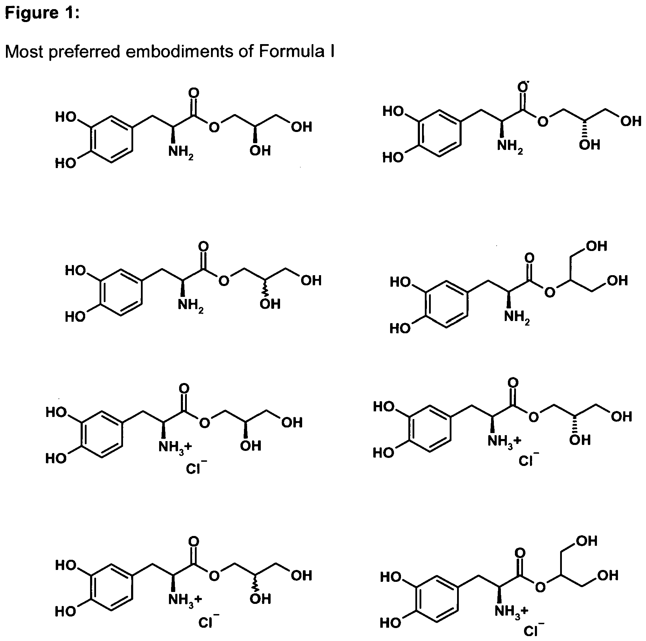

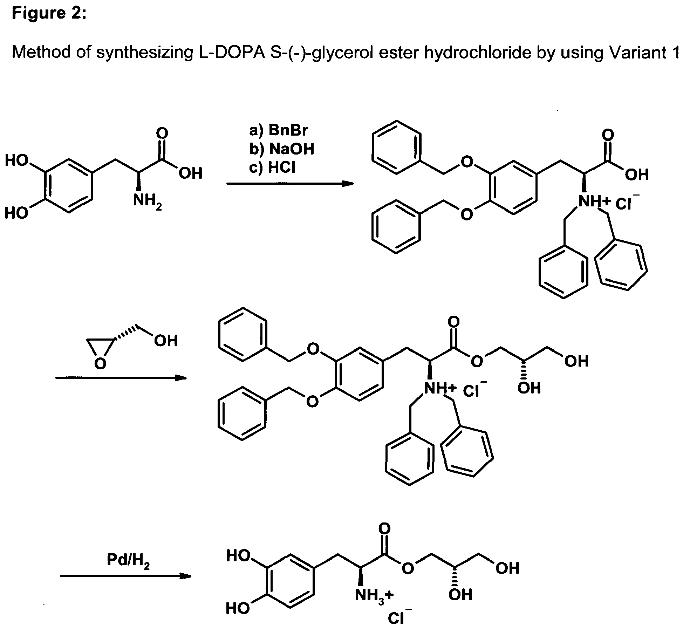

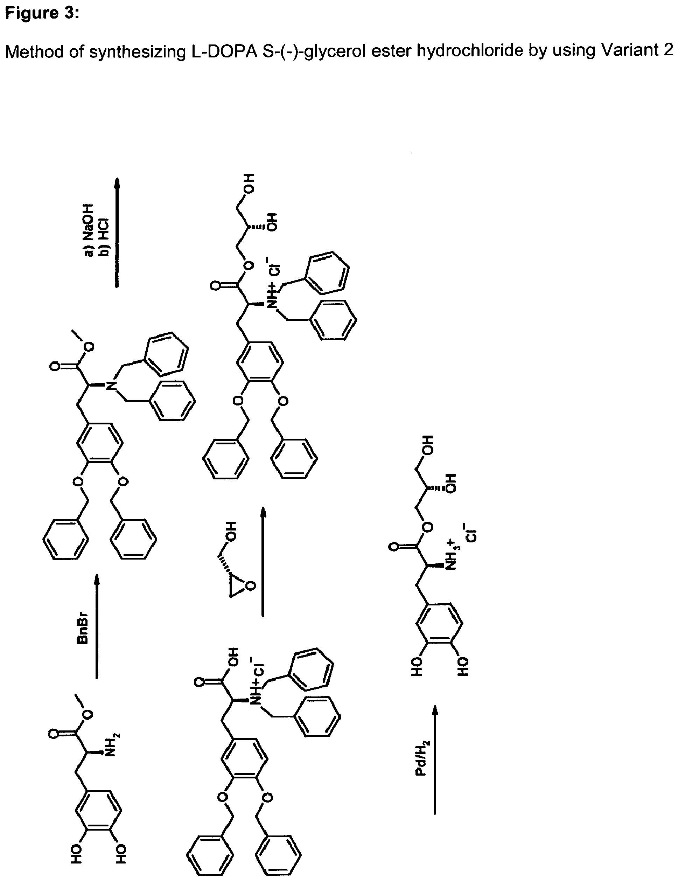

## Claims

1. A Compound having general Formula I 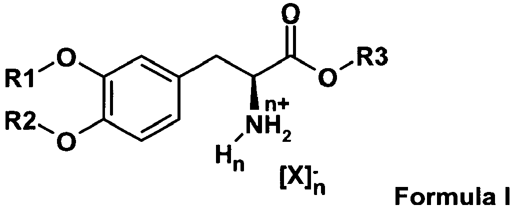
wherein [X]⁻ is a physiologically compatible anion,
wherein n is 0 or 1,
wherein R1 and R2 are, independently of each other, selected from the group comprising hydrogen, hydrogensulfate, phosphate, hydrogen phosphate, dihydrogen phosphate, benzoate, formate, acetate, propionate, butanoate, valerate, silyl,
or
R1, R2 are both hydrogen phosphate, sulfate, methylene, isopropylidene,
wherein R3 represents a residue 1,3-dihydroxyprop-2-yl, (+)-2,3-dihydroxy-prop-1-yl, (-)-2,3-dihydroxyprop-1-yl or *rac*.-2,3-dihydroxyprop-1-yl.

2. A Compound according to claim 1 in which the physiologically compatible anion [X]⁻ is selected from the group consisting of halogenide, pseudohalogenide, sulfate, hydrogensulfate, phosphate, hydrogen phosphate, dihydrogen phosphate, carboxylate like benzoate, formate, acetate, propionate, butanoate, valerate, myristate, octoate, stearate, ascorbate, trifluoracetate, phosphonate, phosphoric acid ester, sulfonate (e.g. mesylate, tosylate) or sulfuric acid ester (e.g. methyl sulfate).

3. A Compound of formula I according to claim 1 or 2 wherein [X]⁻ is Cl⁻, n is 1 and R1 and R2 are both hydrogen.

4. Method of preparing a compound having general Formula I according to claims 1-3, comprising the steps of esterification of a L-DOPA derivative carrying protective groups of the amino and phenolic L-DOPA functionalities with an (un)protected glycerol derivative, and the selective removing of the protective groups subsequently.

5. Method of preparing a compound having general Formula I according to claims 1-3, comprising the steps of coupling a L-DOPA derivative carrying protective groups of amino and phenolic L-DOPA functionalities with an rac.-, R-(+)- or S-(-)-glycidol, whereby the ring of said epoxide is opened, and the selective removing of the protective group subsequently.

6. Method of preparing a compound having general Formula I according to claims 4-5, wherein the L-DOPA ester is obtained by the following steps comprising:
(a) Introduction of suitable protective groups to protect the amino and phenolic functionalities of L-DOPA.
(b) Esterification of the carboxylic group of L-DOPA derivative containing protective groups of amino and phenolic functionalities
(c) Subsequent cleavage of the protective groups from step (a)

7. Method of preparing a compound having general Formula I according to claims 4-6, wherein the protective groups are selected from the group comprising of tert.-butoxycarbonyl (Boc), silyl, carboxybenzyl (Cbz), benzyl, fluorenyloxycarbonyl (Fmoc), trityl, acetonide.

8. Method of preparing a compound having general Formula I according to claim 4-7, wherein said compound is purified by crystallization or preparative chromatographic methods after said preparation.

9. Method of preparing a compound having general Formula I according to claim 4-8, wherein said compound is produced regio-selectively and / or stereoselectively.

10. A composition comprising the compound having the general Formula I according to claims 1-3 and one or more pharmaceutical acceptable excipients for pharmaceutical use.

11. A composition according to claim 10 in which the one or more functional pharmaceutical excipients are selected from like antioxidants, stabilizer, antimicrobials for therapeutic use.

12. A composition according to claims 10 or 11 for use in a method of treatment of neurological disorders, preferably neurodegenerative disorders, such as DOPA-receptor related disorders, in particular Parkinson's disease.

13. A composition according to claims 10 or 11 for use in a method of treatment of neurological disorders according to claim 12, wherein said composition is administered in dosages of between 100 mg and 700 mg per day over 3 to 5 days in a substantially continuous manner.

14. A composition according to claims 10 or 11 for use in a method of treatment of neurological disorders according to claim 13, wherein said composition is administered parenterally.

15. A composition according to claims 10 or 11 for use in a method of treatment of neurological disorders according to claim 14, wherein said composition is administered parenterally via a patch pump.

## Patentansprüche

1. Verbindung mit der allgemeinen Formel I 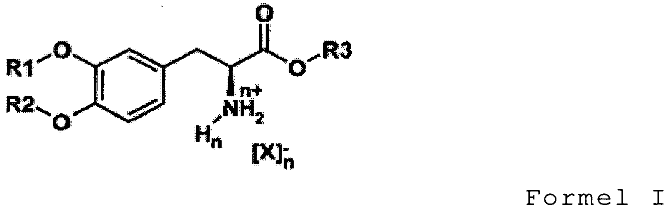
wobei [X]⁻ ein physiologisch kompatibles Anion ist,
wobei n 0 oder 1 ist,
wobei R1 und R2 unabhängig voneinander ausgewählt sind aus der Gruppe bestehend aus Wasserstoff, Hydrogensulfat, Phosphat, Hydrogenphosphat, Dihydrogenphosphat, Benzoat, Format, Acetat, Propionat, Butanoat, Valerat, Silyl,
oder
R1, R2 beide Hydrogenphosphat, Sulfat, Methylen, Isopropyliden sind,
wobei R3 einen Rest 1,3-Dihydroxyprop-2-yl, (+)-2,3-Dihydroxy-prop-1-yl, (-)-2,3-dihydroxyprop-1-yl oder *rac*-2,3-Dihydroxyprop-1-yl darstellt.

2. Verbindung nach Anspruch 1, wobei das physiologisch kompatible Anion [X]⁻ ausgewählt ist aus der Gruppe bestehend aus Halogenid, Pseudohalogenid, Sulfat, Hydrogensulfat, Phosphat, Hydrogenphosphat, Dihydrogenphosphat, Carboxylat wie z.B. Benzoat, Format, Acetat, Propionat, Butanoat, Valerat, Myristat, Octoat, Stearat, Ascorbat, Trifluoracetat, Phosphonat, Phosphorsäureeester, Sulfonat (z.B. Mesylat, Tosylat) oder Schwefelsäureester (z.B. Methylsulfat).

3. Verbindung mit der Formel I nach Anspruch 1 oder 2, wobei [X]⁻ Cl⁻ ist, n 1 ist und R1 und R2 beide Wasserstoff sind.

4. Verfahren zur Herstellung einer Verbindung mit der allgemeinen Formel I nach den Ansprüchen 1 bis 3, umfassend die Schritte der Veresterung eines L-DOPA-Derivats, das Schutzgruppen der Amino-und Phenol-L-DOPA-Funktionalitäten mit einen (un)geschützten Glycerol-Derivat trägt, und danach des selektiven Entfernens der Schutzgruppen.

5. Verfahren zur Herstellung einer Verbindung mit der allgemeinen Formel I nach den Ansprüchen 1 bis 3, umfassend die Schritte des Koppelns eines L-DOPA-Derivats, das Schutzgruppen der Amino- und Phenol-L-DOPA-Funktionalitäten trägt, mit einem rac.-, R-(+)- oder S-(-)-Glycidol, wodurch der Ring des Epoxids geöffnet wird, und danach des selektiven Entfernens der Schutzgruppen.

6. Verfahren zur Herstellung einer Verbindung mit der allgemeinen Formel I nach den Ansprüchen 4 bis 5,
wobei der L-DOPA-Ester durch die folgenden Schritte erhalten wird:
(a) Einbringen von geeigneten Schutzgruppen, um die Amino- und Phenol-L-DOPA-Funktionalitäten von L-DOPA zu schützen,
(b) Veresterung der Carboxylgruppe des L-DOPA-Derivats, das Schutzgruppen der Amino- und Phenol-Funktionalitäten enthält,
(c) danach Spaltung der Schutzgruppen aus Schritt (a).

7. Verfahren zur Herstellung einer Verbindung mit der allgemeinen Formel I nach den Ansprüchen 4 bis 6, wobei die Schutzgruppen ausgewählt sind aus der Gruppe bestehend aus tert.-Butoxycarbonyl (Boc), Silyl, Carboxybenzyl (Cbz), Benzyl, Fluorenyloxycarbonyl (Fmoc), Trityl, Acetonid.

8. Verfahren zur Herstellung einer Verbindung mit der allgemeinen Formel I nach Anspruch 4 bis 7, wobei die Verbindung durch Kristallisation oder präparative chromatographische Verfahren nach der Herstellung aufgereinigt wird.

9. Verfahren zur Herstellung einer Verbindung mit der allgemeinen Formel I nach Anspruch 4 bis 8, wobei die Verbindung regio-selektiv und/oder stereo-selektiv erzeugt wird.

10. Zusammensetzung, umfassend die Verbindung mit der allgemeinen Formel I nach den Ansprüchen 1 bis 3 und einen oder mehrere pharmazeutisch akzeptable Träger zur pharmazeutischen Verwendung.

11. Zusammensetzung nach Anspruch 10, wobei der eine oder die mehreren funktionalen pharmazeutischen Träger ausgewählt sind aus Antioxidantien, Stabilisatoren, antimikrobiellen Substanzen zur therapeutischen Verwendung.

12. Zusammensetzung nach den Ansprüchen 10 oder 11 zur Verwendung bei einem Verfahren zur Behandlung von neurologischen Störungen, bevorzugt neurodegenerativen Störungen, wie z.B. mit dem DOPA-Rezeptor verbundenen Störungen, insbesondere der Parkinson-Krankheit.

13. Zusammensetzung nach den Ansprüchen 10 oder 11 zur Verwendung bei einem Verfahren zur Behandlung von neurologischen Störungen nach Anspruch 12, wobei die Zusammensetzung in Dosierungen zwischen 100 mg und 700 mg pro Tag über 3 bis 5 Tage in einer im Wesentlichen durchgehenden Weise verabreicht wird.

14. Zusammensetzung nach den Ansprüchen 10 oder 11 zur Verwendung bei einem Verfahren zur Behandlung von neurologischen Störungen nach Anspruch 13, wobei die Zusammensetzung parenteral verabreicht wird.

15. Zusammensetzung nach den Ansprüchen 10 oder 11 zur Verwendung bei einem Verfahren zur Behandlung von neurologischen Störungen nach Anspruch 14, wobei die Zusammensetzung parenteral über eine Patch-Pumpe verabreicht wird.

## Revendications

1. Composé de la formule générale I 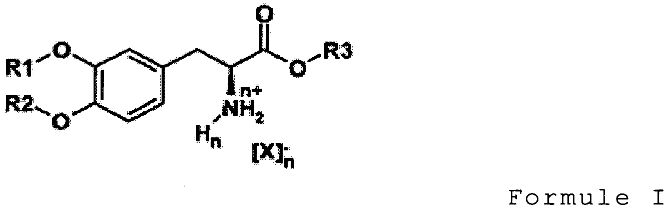
dans lequel [X]⁻ est un anion physiologiquement compatible,
dans lequel n est 0 ou 1,
dans lequel R1 et R2 sont, indépendamment l'un de l'autre, choisis à partir du groupe consistant en hydrogène, hydrogénosulfate, phosphate, hydrogénophosphate, dihydrogénophosphate, benzoate, formate, acétate, propionate, butanoate, valérate, silyle,
ou
R1, R2 sont tous deux de l'hydrogénophosphate, du sulfate, du méthylène, de l'isopropylidène,
dans lequel R3 représente un résidu 1,3-dihydroxyprop-2-yl, (+)-2,3-dihydroxy-prop-1-yl, (-)-2,3-dihydroxyprop-1-yl ou rac-2,3-dihydroxyprop-1-yl.

2. Composé selon la revendication 1, dans lequel l'anion [X]- physiologiquement compatible est choisi à partir du groupe consistant en halogénure, pseudohalogénure, sulfate, hydrogénosulfate, phosphate, hydrogénophosphate, dihydrogénophosphate, carboxylate comme p.ex. benzoate, formate, acétate, propionate, butanoate, valérate, myristate, octoate, stéarate, ascorbate, trifluoracétate, phosphonate, esters de l'acide phosphorique, sulfonate (p.ex. mésylate, tosylate) ou esters de l'acide sulfurique (p.ex. méthylsulfate).

3. Composé de la formule I selon la revendication 1 ou 2, dans lequel [X]- est Cl-, n est 1 et R1 et R2 sont tous deux de l'hydrogène.

4. Procédé de préparation d'un composé de la formule générale I selon les revendications 1 à 3, comprenant les étapes d'estérifier un dérivé de L-DOPA porteur de groupes protecteurs des fonctionnalités amino et phénoliques de L-DOPA avec un dérivé de glycérol (non)protégé, et puis enlever de façon sélective les groupes protecteurs.

5. Procédé de préparation d'un composé de la formule générale I selon les revendications 1 à 3, comprenant les étapes d'accoupler un dérivé de L-DOPA porteur de groupes protecteurs des fonctionnalités amino et phénoliques de L-DOPA à un rac.-, R-(+)- ou S-(-)-glycidol, par quoi l'anneau de cet époxyde est ouvert, et puis enlever de façon sélective le groupe protecteur.

6. Procédé de préparation d'un composé de la formule générale I selon les revendications 4 à 5,
dans lequel l'ester de L-DOPA est obtenu par les étapes suivantes, comprenant:
(a) l'introduction des groupes protecteurs propres à protéger les fonctionnalités amino et phénoliques de L-DOPA,
(b) l'estérification du groupe carboxylique du dérivé de L-DOPA comportant des groupes protecteurs de fonctionnalités amino et phénoliques,
(c) puis le clivage des groupes protecteurs à partir de l'étape (a).

7. Procédé de préparation d'un composé de la formule générale I selon les revendications 4 à 6, dans lequel les groupes protecteurs sont choisis à partir du groupe consistant en tert.-butoxycarbonyle (Boc), silyle, carboxybenzyle (Cbz), benzyle, fluorényloxycarbonyle (Fmoc), trityle, acétonide.

8. Procédé de préparation d'un composé de la formule générale I selon la revendication 4 à 7, dans lequel ce composé est purifié par cristallisation ou procédés chromatographiques préparatifs après cette préparation.

9. Procédé de préparation d'un composé de la formule générale I selon la revendication 4 à 8, dans lequel ce composé est produit de façon régio-sélective et/ou stéréo-sélective.

10. Composition comprenant le composé de la formule générale I selon les revendications 1 à 3 et un ou plusieurs excipients pharmaceutiquement acceptables pour l'utilisation pharmaceutique.

11. Composition selon la revendication 10, dans lequel l'un ou les plusieurs excipients pharmaceutiques fonctionnels sont choisis à partir des antioxydants, stabilisateurs, agents antimicrobiens, pour l'utilisation thérapeutique.

12. Composition selon les revendications 10 ou 11 pour l'utilisation dans un procédé de traitement des affections neurologiques, de préférence affections neuro-dégéneratives, comme p.ex. des affections associées au récepteur DOPA, en particulier la maladie de Parkinson.

13. Composition selon les revendications 10 ou 11 pour l'utilisation dans un procédé de traitement des affections neurologiques selon la revendication 12, dans lequel cette composition est administrée en des dosages d'entre 100 mg et 700 mg par jour pendant 3 à 5 jours de façon essentiellement continue.

14. Composition selon les revendications 10 ou 11 pour l'utilisation dans un procédé de traitement des affections neurologiques selon la revendication 13, dans lequel cette composition est administrée par voie parentérale.

15. Composition selon les revendications 10 ou 11 pour l'utilisation dans un procédé de traitement des affections neurologiques selon la revendication 14, dans lequel cette composition est administrée par voie parentérale en utilisant une pompe patch.
